# EUROPEAN PATENT APPLICATION

(11) **EP 2 278 300 A1**
(43) Date of publication of application: **26.01.2011**
(21) Application number: 09166112.4
(22) Date of filing: 22.07.2009
(51) Int. Cl.: G01N 21/45, G01N 21/78, G01N 33/553, G01N 33/12

(54) **Device comprising a polymer layer and a reflecting layer**

(71) Applicant: Universität Wien, 1010 Wien (AT)
(72) Inventor: Pittner, Fritz, 1230 Wien (AT); Ibrisimovic, Nadira, 1050 Wien (AT); Ibrisimovic, Mirza, 1050 Wien (AT); Barth, Margit, 1230 Wien (AT); Bohrn, Ulrich, 2486 Pottendorf (AT)
(74) Representative: Bühler, Dirk

(57) **Abstract**

The present invention relates to a device comprising a reflecting layer and a biodegradable polymer layer. Such a device may be used as sensor for the analysis of natural products such as pharmaceutical products and cosmetics.

## Description

### FIELD OF THE INVENTION

The present invention *inter alia* relates to a device comprising a polymer layer and a reflecting layer and to its preparation. Since the device is capable of indicating the presence of biomolecules (derived e.g. from bacteria) by a colour change, the invention furthermore relates to the use of said device as sensor for determining e.g. the quality of natural products such as cosmetics or pharmaceutical products.

### BACKGROUND OF THE INVENTION

There is a wide variety of natural products, which are offered to consumers. Such natural products do not only comprise foods of any kind but also cosmetics and pharmaceutical products.

Even if natural products are produced, handled and stored according to the official guidelines for the mentioned steps, the period, during which they can be used by consumers, is limited due to spoilage of the natural products. For this reason, so called expiration dates are typically assigned to natural products.

However, expiration dates are not necessarily reliable factors since the quality of natural products seems to vary to a large extent. Furthermore, in case of improper handling (e.g. during production, transportation or storage), spoilage of the natural product may occur prior to the expiration date given.

Spoilage of natural products can mainly be attributed to microorganisms, which "live on the natural product", i.e. which are able to utilize and/or metabolize certain components and/or molecules of the natural product. In this regard, the degradation of components and/or molecules seems to represent an essential step towards usage of said components by the microorganisms.

In order to be able to use the components, microorganisms usually excrete enzymes with degrading activities in order to degrade said components. Thus, the degradation is a catalyzed process. Said enzymes do not seem to be highly specific since they have to be able to "work" in a wide substrate range.

Since the consumer is not able to detect the microorganisms associated with the natural product simply by looking at the product, there is the risk of using a contaminated natural product and accordingly of being infected by said associated microorganisms in case the immune system is unable to handle the number and/or aggressiveness of said microorganisms.

Thus, there is the need for a device indicating the quality of a natural product "in real time", i.e. at that point in time, when the consumer is looking at the product and not only after several days upon incubation of the product in order to test for spoilage-causing microorganisms (a standard test, which can be done in order to analyze spoilage by microorganisms). Such a device should have a simple setup. Furthermore, the device should be cost-efficient in terms of the materials needed. Clearly, any risk of contaminating the natural product by the device (in terms of e.g. harmful chemicals or the like) needs to be avoided. Finally, the production of such a device should be easy, fast and cost-efficient.

Thus, there is a need for a cost-efficient "real time" sensor indicating the quality of a natural product which can be produced simply (e.g. in terms of materials needed) and fast.

### OBJECTS AND SUMMARY OF THE INVENTION

It is one object of the present invention to provide a device comprising a biodegradable polymer layer positioned on a reflecting layer. Said device is configured such that biomolecules capable of degrading said polymer layer are allowed to contact said polymer layer. Furthermore, said device is configured such that degradation of said polymer layer results in a colour change. Said device does not comprise an additional reflecting layer; thus, said device comprises only one reflecting layer.

It is among the further objects of the present invention to provide a method of preparing an above-mentioned device and to provide a method of analyzing the age and/or quality of a natural product. The use of a device as described above for the analysis of the age and/or quality of a natural product is also among the further objects.

These and other objects as they will become apparent from the ensuing description are attained by the subject matter of the independent claims. The dependent claims relate to some of the preferred embodiments of the present invention.

The inventors have surprisingly found that a two-layer-setup comprising only a polymer layer and a reflecting layer can be used as sensor indicating the presence of spoilage-causing microorganisms; thus, a device comprised of said two layers only can be used in order to analyze the quality and/or age of natural products comprising foods, medical products and cosmetics.

As mentioned above, the invention is in the first aspect concerned with a device comprising a biodegradable polymer layer positioned on a reflecting layer, wherein the device is configured such that biomolecules capable of degrading said polymer layer are allowed to contact said polymer layer, wherein the device is configured such that degradation of said polymer layer results in a colour change and wherein said device comprises only one reflecting layer.

In a preferred embodiment of the present invention, said polymer layer is optically transparent.

In another preferred embodiment of the present invention, said colour change is a change in the intensity of a colour.

In another preferred embodiment of the present invention, said device is configured such that degradation of said polymer layer results in a colour change and/or change of intensity of the reflected light.

In yet another preferred embodiment of the present invention, said colour change is visible to the human eye and/or can be detected using an optical measuring device. In still another preferred embodiment of the present invention, said biomolecules comprise enzymes and/or catabolic metabolites of microorganisms and/or of the natural product.

The degradation of said polymer layer may *inter alia* be expressed as a change in the thickness and/or as a change in the refractive index of said polymer layer. A change in the thickness of said polymer layer may also be due to shrinking or swelling of the polymer layer as a result from degradation.

Thus, in a preferred embodiment of the first aspect of the invention, said device is configured such that the change in the thickness of said polymer layer results in a colour change visible.

In another preferred embodiment of the first aspect of the invention, said device is configured such that a change in the refractive index of said polymer layer results in a colour change.

Furthermore, in another preferred embodiment of the first aspect of the invention, said device is configured such that a change in the thickness and in the refractive index of said polymer layer results in a colour change.

In an also preferred embodiment of this first aspect of the present invention, said polymer layer does not comprise a colouring agent. Thus, in this embodiment, said polymer layer does not comprise a dye and/or a pigment selected from the group of food colouring, acryl-dyes, azo-dyes, fluorescence-dyes and luminescence-dyes.

In another preferred embodiment of this aspect of the present invention, said polymer layer does not comprise any nutrients and/or reactive chemicals. Preferably, said polymer layer consists of a biodegradable polymer and a cross-linking agent only. Furthermore, as mentioned above, said device does not comprise a second reflecting layer or any further additional reflecting layer. Said additional reflecting layer not comprised in the device according to the invention may be any reflecting layer, e.g. a mirror layer or a semi-reflective layer.

In another preferred embodiment, the device consists of a polymer layer and a reflecting layer.

In a further preferred embodiment of this aspect of the present invention, said polymer layer is selected from a polymer comprising poly(lactic-co-glycolic acid), polylactic acid, poly-L-lactic acid, polyhydroxybutyrate and polyvinylcaprolactame.

In an also preferred embodiment of this first aspect of the invention, said polymer layer additionally comprises a cross-linking agent, wherein said cross-linking agent is preferably Desmodur.

In an also preferred embodiment of the first aspect of the present invention, said reflecting layer is selected from a material comprising a metal, preferably gold, titan or aluminium; an alloy, preferably CrNi or TiAl; silicium; a reflecting oxide, preferably Eloxal; glassy carbon; tin; Geberit; opaque reflecting glass; a lacquer; and a metallic or coloured pigment within a support.

In an even more preferred embodiment of the first aspect of the present invention, said reflecting layer is selected from a material consisting of glassy carbon or silicium.

In still another preferred embodiment of the first aspect of the invention, said reflecting layer corresponds to or is part of the packaging material of the natural product.

In a further preferred embodiment of this aspect of the invention, said reflecting layer is a mirror layer, which is remitting the incident light completely. Said mirror layer is preferably made of a material comprising silicium, a reflecting oxide such as Eloxal, glassy carbon and tin. Said mirror layer may be coloured or uncoloured.

In another preferred embodiment of the first aspect of the invention, said reflecting layer is a semi-reflecting layer, which is remitting the incident light to some extent only. Preferably, said semi-reflecting layer remits incident light to about 95%, about 90%, about 85%, about 80%, about 75%, about 70%, about 60%, about 50% or about 40%. Said semi-reflecting layer is preferably selected from a material comprising Geberit, opaque reflecting glass, a lacquer and a metallic or coloured pigment within a support layer. Said semi-reflecting layer may be coloured or uncoloured. Preferably, said semi-reflecting layer displays a bright colour, if coloured.

In still another preferred embodiment of the first aspect of the invention, said reflecting layer has a thickness of about 1 nm to about 1000 nm. The reflecting layer may also be about 10 nm to about 100 nm thick.

In another preferred embodiment, said polymer layer has a thickness of about 5 nm to about 1000 nm prior to being contacted by said biomolecules.

In yet another preferred embodiment of the first aspect of the present invention, said device comprises a support layer onto which said reflecting layer is positioned. In certain embodiments, said support layer is selected from a material selected from the group comprising polyethylenterephtalate, polyamide, glass, polyethylene, polycarbonate, polypropylene, silicone and ceramics. Said support layer may have a thickness of about 10 nm to about 5000 µm.

Thus, in another preferred embodiment of the present invention, the device consists of a support layer, a reflecting layer and a polymer layer.

In yet another preferred embodiment of the first aspect of the present invention, said device comprises a reflecting layer comprising e.g. metallic or coloured pigments and a support for said pigments, wherein said support is preferably selected from a material given for the support layer as set out above. For this embodiment, it needs to be understood that such a "mixed" layer is defined as reflecting layer.

In still another preferred embodiment of the present invention, the biomolecules mentioned in the first aspect above are allowed to penetrate said reflecting layer and/or said support layer in order to contact said polymer layer.

In yet another preferred embodiment, the device as described in the first aspect above also comprises a reference device.

The present invention is in a second aspect concerned with a method for preparing a device of the first aspect of the invention. Said method comprises at least the steps of
a) Providing a reflecting layer, and
b) Applying a biodegradable polymer layer onto said reflecting layer.

In case a support layer is present, the present invention relates in a preferred embodiment of this second aspect of the invention to a method comprising at least the steps of
a) Providing a support layer,
b) Applying a reflecting layer onto said support layer, and
c) Applying a biodegradable polymer layer onto said reflecting layer.

In an especially preferred embodiment of this second aspect of the invention, said polymer layer is applied by dip coating or film-printing.

In a third aspect, the present invention relates to a method for analyzing the age and/or quality of a natural product comprising cosmetics, pharmaceutical products and foods. Said method comprises at least the following steps:
a) Providing a device according to the first aspect of the invention;
b) Contacting said device with said natural product;
c) Determining the colour of said device;
d) Comparing the colour of said device to the colour of a reference device;
e) Determining the age and/or quality of said natural product according to this comparison.

In a preferred embodiment of the method of the third aspect of the invention, said polymer layer of the device according to the first aspect of the invention is being contacted in step b) directly with said natural product.

In another preferred embodiment of the method of the third aspect of the invention, the reflecting layer and/or the support layer, if present, of the device according to the first aspect of the invention is/are being contacted in step b) with said natural product such that biomolecules are allowed to penetrate the reflecting layer and/or the support layer in order to contact said polymer layer.

In another preferred embodiment of the method of the third aspect of the invention, the colour of the device is determined in step c) by looking at the device and/or by using an optical measuring device.

The present invention is in yet another aspect concerned with the use of a device according to the first aspect for the analysis of the age and/or quality of a natural product comprising cosmetics, pharmaceutical products and foods. The analysis of cosmetics and pharmaceutical products may be preferred.

In a preferred embodiment of said aspect, the invention relates to the use of a device according to the first aspect of the invention for the analysis of the age and/or quality of a natural product by detecting microorganisms present in the natural product.

In an even more preferred embodiment of this aspect, the present invention relates to the use of a device according to the first aspect of the invention for the analysis of the age and/or quality of a natural product by detecting enzymes and/or catabolic metabolites of microorganisms and/or of the natural product via the degradation of said polymer layer.

### DESCRIPTION OF THE FIGURES

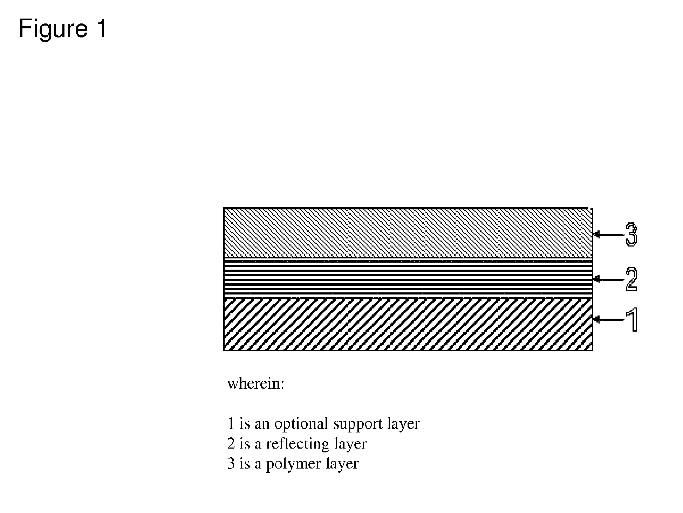

Fig. 1: Figure 1 schematically depicts a device according to the invention. Layer 1 represents the optional support layer, whereas layer 2 represents the reflecting layer and layer 3 represents the biodegradable polymer layer.

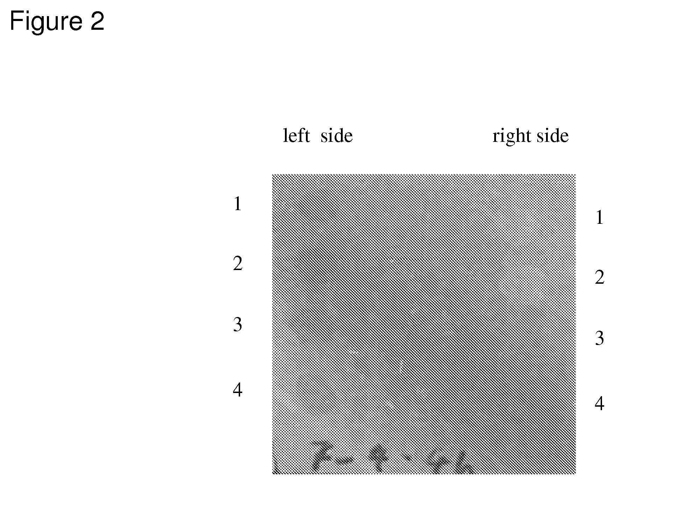

Fig. 2: Figure 2 shows a device according to the invention, which has been incubated with different concentrations of an enzyme mix comprised of different proteases. The device was incubated on spots 1 to 4 on the left side and 1 to 2 on the right side with different concentrations of the mix; spots 3 and 4 on the right side were incubated with control solutions.

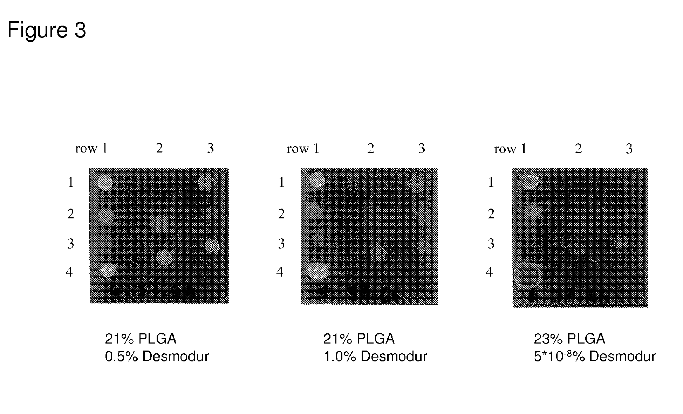

Fig. 3: Figure 3 depicts three devices according to the invention with different PLGA and Desmodur concentrations, respectively. The three devices were incubated according to an identical pipetting scheme given in the example section of the specification with different samples, namely meat juice, meat homogenate, bacteria, solution for contact lenses (contaminated) and hand creme (contaminated).

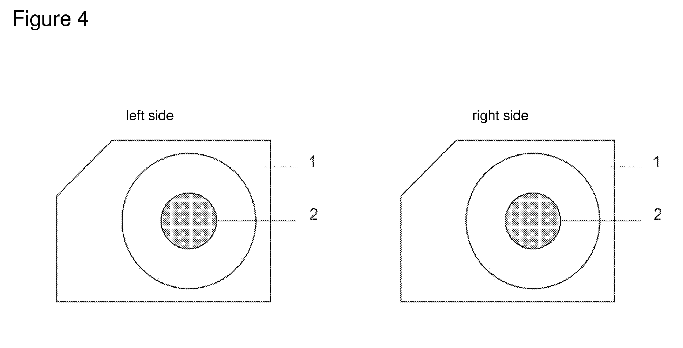

Fig. 4: Figure 4 schematically depicts one exemplary way of using a device according to the invention for determining the contamination state of a contact lens solution (1: lens container; 2: sensor according to the invention on the bottom of the box):
   left side: uncontaminated contact lense solution (sensor 2 is blue);
   right side: bacterially contaminated contact lense solution (sensor 2 is red).

### DETAILED DESCRIPTION OF THE INVENTION

As already set out above, the present invention partially resides in the surprising finding that a two-layer-setup comprising a polymer layer and a reflecting layer can be used as sensor. The inventors have surprisingly found that a device comprised of said two layers only can be used for the analysis of the quality and/or age of natural products comprising medical products, cosmetics and foods.

In the following, definitions important for the understanding of the present invention are given and the defined terms are described in combination with further preferred embodiments.

As used in the specification and the claims, the singular forms of "a" and "an" also include the corresponding plurals unless the context clearly dictates otherwise.

The terms "about" and "approximately" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising of'. If hereinafter a group is defined to comprise at least a certain number of embodiments, this is also meant to encompass a group which preferably consists of these embodiments only.

The term "biodegradable polymer layer" as used herein defines a layer, which comprises a biodegradable polymer.

The term "polymer" as used herein may in general be understood as referring either to a naturally occurring polymer (e.g. as present in or secreted by microorganisms such as agarose in algae) or a synthetic polymer resulting e.g. from a synthesis reaction comprising *inter alia* monomer units of the polymer and a cross-linking agent. A naturally-occurring polymer may also be referred to as "biopolymer". Thus, a synthetic polymer according to the definition cannot be found in nature in exactly the same condition and/or modification and/or conformation. However, every naturally occurring polymer (biopolymer) which has been subjected to modification (such as e.g. cross-linking) resulting in conditions and/or conformations which are not naturally occurring has been transformed into a synthetic polymer and is thus no biopolymer any more by consequence. Thus, a modified biopolymer can be classified as synthetic polymer if its state of modification is not found in nature. Preferably, polymers used in the present invention and described in further detail below belong to the class of synthetic polymers as defined above.

"Biodegradable polymer" as used herein defines a polymer, wherein the stability of the polymer is influenced by biomolecules as defined below, i.e. the biomolecules are capable of degrading said polymer layer. Typically, said degradation occurs via the cleavage of covalent bonds within the polymer. Thus, the process of degradation is irreversible resulting in a definite "signal" of the device of the present invention in form of a irreversible colour change.

For the setup of the system it needs to be understood that the thickness of the biodegradable polymer layer correlates with its degradation time. The thicker the biodegradable polymer layer, the longer it takes the biomolecules described below to degrade said layer. By adjusting the thickness of the biodegradable polymer layer, the sensitivity over time of the device may thus be adjusted.

Preferably, the biodegradable polymer layer is a "biomimetic" polymer, i.e. a polymer with characteristics mimicking a natural material. A "natural material" is meant to describe substances found *in vivo* and/or in natural products; thus, the advantage of a biomimetic polymer resides in the fact that any potential risk of contaminating a natural product to be tested due to toxic or so far uncharacterized ingredients and/or molecules is excluded.

Most preferably, the biodegradable polymer is comprised of a biomimetic polymer, which has already been approved and extensively tested for its behaviour when exposed to a natural product and/or a living organism. In this respect, it should be mentioned that poly(lactic-co-glycolic acid) (PLGA), which may preferably be used as biodegradable polymer, is indeed approved for *in vivo* use (PLGA is approved and used as coating for stents in the medical field) and thus represents a biomimetic polymer as defined herein.

Thus, in an especially preferred embodiment of the present invention, said polymer layer comprises poly(lactic-*co*-glycolic acid) (PLGA) as polymer.

Further polymers, which can be used according to the present invention, may be selected from the group of polymers comprising polylactic acid (PLA), poly-L-lactic acid (PLLA), polyhydroxybutyrate (PHB) and polyvinylcaprolactame (PVCL) or any other polymer, which falls under the classification of a polymer degradable by biomoleculesas defined above. Preferably, biodegradable synthetic polymers can be used. This may also comprise synthetic polymers of gelatine, agarose, dextrose, lipids, cellulose, starch, chitin, polyhydroxyalkanoates, poly(-caprolactone) (PCL) or PCL-systems, poly(ethylene/butylenes) succinate or poly(ethylene/butylenes) adipate or polynucleic acids.

As mentioned above, the biodegradable polymer layer may additionally comprise a cross-linking agent. This cross-linking agent may be a bifunctional agent, such as e.g. diisocyanat, glutardialdehyde or Desmodur (Desmodur 2460 M, Bayer). Desmodur products based on diphenylmethane diisocyanate (MDI), toluene diisocyanate (TDI), hexamethylene diisocyanate (HDI) and isophorone diisocyanate (IPDI) may also be used.

As explained in detail below, the colour of the device of the present invention is defined by remission of incident light by the reflecting layer in combination with the properties of the polymer layer. Thus, the polymer layer may display different characteristics as long as it is biodegradable and optically transparent such that incident light is able to contact the reflecting layer and remitted light is able to pass the polymer layer (wherein the polymer layer does, however, influence the remission).

Preferably, the polymer layer does not comprise a colouring agent from the group of colouring agents comprising dyes and pigments as set out above since said colouring agent can be a potential risk to the natural products. However, it needs to be understood that the polymer layer itself may display a colour, which is due to its nature (i.e. its chemical composition) and not due to the addition of any further agent. Thus, "uncoloured" as used herein with respect to the polymer layer is intended to describe that no external colouring agent has been added to the polymer.

Furthermore, the polymer layer may not comprise any nutrients and/or reactive chemicals and/or any further materials or compounds, but may only be comprised of said biodegradable polymer and preferably a cross-linking agent.

The term "biomolecule" as used herein refers to molecules capable of degrading the polymer layer as defined above. In most cases, said biomolecules will be enzymes secreted from either the natural product to be tested or from a microorganism associated with said natural product or from both. Examples for such enzymes are phospholipases, pronases, proteinases, hydrolases, lipases, esterases. Biomolecules according to the present invention also comprise any molecules present in or secreted from the natural product to be analysed and/or present in or secreted from any further object associated with said natural product, e.g. a microorganism, of non-enzymatic origin which are capable of degrading the biodegradable polymer layer, i.e. for example intermediates of the metabolism of such microorganisms associated with said natural product. Such non-enzymatic molecules are defined in the context of the invention as catabolic metabolites. Examples for such catabolic metabolites comprise volatile acids, volatile bases, volatile aldehydes, volatile mercaptans and sulfur compounds. Said molecules may also result from aging processes of the natural product.

The term "reflecting layer" as used herein describes a layer comprising a reflecting material.

In addition to the materials set out above, said reflecting layer may be selected from any organic or inorganic material capable of reflecting light in an amount visible to the human eye.

"Reflecting material" may be any material capable of either remitting incident light completely (also termed "mirror" when used herein) or to some extent only. Incident light may be a natural light source or any light source known including regular light bulbs and the like. However, the remission needs to be to such an extent that it is influenced by the properties of the polymer layer and works according to the setup of the system, i.e. that degradation of the polymer layer results in a colour change. Preferably, the material used for the reflecting layer is inert to any reactions with the natural product. The reflecting layer may be configured such that biomolecules as defined above are able to penetrate said layer in order to contact the polymer layer on the other side of the reflecting layer.

In one embodiment, the nature of the reflecting layer may be described as being "continuous". The term "continuous" with respect to the reflecting layer means that the reflecting layer does not comprise any island-like isolated structures or a plurality of discrete islands or a layer of islands wherein the islands are structured in a more or less regular arrangement. In no case, however, the term "continuous" means that the reflecting layer is not permeable to biomolecules as defined above. The reflecting layer may be made of an electrically conductive material. Therefore, in a further preferred embodiment, the continuous reflecting layer as described above is also conductive. Thus, such a layer may be described as a "continuous and conductive reflecting layer".

In another embodiment, the nature of the reflecting layer may be described as being "island-like", i.e. the reflecting layer comprises structures selected from *inter alia* island-like isolated structures or a plurality of discrete islands or a layer of islands wherein the islands are structured in a more or less regular arrangement. In this embodiment, a further support layer is present, onto which the reflecting material is positioned in an "island-like" fashion as set out above. This can e.g. be the case for metallic or coloured pigments onto a support layer, but also e.g. for opaque reflecting glass or lacquers onto a support layer.

The reflecting layer is preferably uncoloured. However, it may also be coloured with at least one colouring agent selected from colouring agents as set out above, wherein non-toxic and approved colouring agents are preferred for the reasons set out above.

In a preferred embodiment, the biodegradable polymer layer is the only layer degradable by biomolecules as defined above. Said biomolecules may penetrate the reflecting layer and contact the biodegradable polymer layer but may not react with the reflecting layer.

The term "natural product" as used herein comprises any product which is subjected to spoilage and used by humans such that it represents a potential health risk, i.e. for example when applied to the outside of the body or ingested. Such a health risk may be associated with aging processes of the natural product resulting in biomolecules as defined above or particularly due to contamination by microorganisms secreting biomolecules as defined above. Such natural products may be medical products or cosmetics, but also foods of any kind. It needs to be understood that the term "natural" is in the present invention used with respect to "natural product" to describe the molecular structure and/or molecular composition of the product, which is degradable by biomolecules as set out above.

Examples for such structures are chemical linkages such as e.g. any ester- or amide-bonds within the product (e.g. in a synthetic polymeric structure of the product), which are biodegradable. Further examples of such structures are modified fatty acids and/or modified proteins comprised in cosmetics, which are biodegradable, resulting in said cosmetics being "natural products" according to the present definition.

Foods are *inter alia* comprised of fish, meat, milk products, vegetables, carbohydrate-containing foods such as bread, and the like. The term "natural product" in the context of this invention does, furthermore, not define a "natural product" in a way that it has to be untreated. The natural product may be treated or untreated. Any natural product according to the invention may be treated and/or e.g. used in preparation processes, such as e.g. sterilization treatments (heating, irradiation), freezing, boiling, and the like. However, even after such steps, the natural product still represents a substrate for spoilage and/or decay processes by e.g. microorganisms. The natural product may also be packaged in any way known to the person skilled in the art.

The term "age and/or quality" relates to the natural product as defined above. As mentioned above, natural products possess different time frames, in which they may be used according to their purposes. A very important aspect is the age of the product because of the correlation of contamination by e.g. microorganisms and time. Generally, the contamination of the natural product increases over time and thus correlates with e.g. storage time. In case of inadequate storage and/or delivery, the process of spoilage may even occur much earlier. The term "quality" in the context of the present invention thus mainly describes whether a medical product may still be used according to its purpose, i.e. whether a cosmetic product may still be applied to the human body or whether a food is still edible.

In the following paragraphs, the mode of operation of the device will be explained.

The system is in general based on the remission of light by a reflecting layer; thus, the colour of the device is firstly dependent on the reflecting layer used and thus the nature of the reflecting material. Furthermore, however, the polymer layer, which is positioned on the reflecting layer, also influences the process of remission. Thus, the colour of the device also depends on the presence of the polymer layer and the optical properties and/or characteristics of the polymer layer.

The change of the colour of the device according to the invention is thus due to the optical setup of the device and not due to a change of the colour of the polymer layer only.

If e.g. a polymer layer with a thickness of 100 nm comprising PLGA is positioned on a reflecting layer, the device will have a specific colour resulting from the cumulative effect of remission and the optical properties of the PLGA polymer. Obviously, the device displays a different colour in case the PLGA layer is absent since the effect is then dependent on the remission by the reflecting layer only. Thus, such a device comprised of a reflecting layer only displays a different colour than the device comprising said polymer layer as well.

Obviously, there are not only "on" and "off" situations for the polymer layer positioned on the reflecting layer, but also intermediate situations. Thus, if the optical properties and/or characteristics of the polymer layer change, the colour of the device will change as well. The intensity/degree of colour change is in this respect proportional to the degree of change in the polymer layer. One major factor influencing the characteristics of the polymer layer is obviously the thickness of the polymer layer; another factor relates to the refractive index of the polymer layer, which is characterizing the optical thickness of the layer. Obviously, said factors may also be referred to in combination.

With respect to the present invention, a "change of colour" may also be formulated as "change in the intensity of a colour" due to the above-described mechanism.

Overall, incident light is remitted by the reflecting layer as described above and ultimately leads in combination with the optical properties of the polymer layer to a specific colour of the device which may appear to the human eye as e.g. a red, white, blue or green colour.

For the present invention, the following exemplary description may furthermore illustrate the mode of operation of the sensor:

A natural product is infested with microorganisms. Said microorganisms excrete enzymes implicated in the "digestion", i.e. the degradation, of the natural product. However, said enzymes will also act on the biodegradable polymer layer of the device; thus, they degrade the biodegradable polymer layer as well. The degree of degradation is depending on the number of enzymes (and thus the number of microorganisms present); the degree of degradation, in turn, is proportional to the colour of the device as set out above. Thus, the colour of the device is indicative for the extent to which the natural product is infested with microorganisms.

It needs to be understood that the mode of operation of the device according to the present invention is not based on interference and/or any phenomena related to interference.

The device according to the invention may be comprised of a combination of devices and thus cover a broad range of reactivity and sensitivity due to the characteristics of each single device. An example for such a device is an embodiment wherein biodegradable polymer layers with differently cross-linked polymers are placed next to each other in such a way, that a range of biodegradable polymer layers is formed and covered, e.g. a polymer layer with a cross-linking range of 0.01 %, next to 0.5%, next to 1.0%, next to 5.0%. Also, a gradient might be formed ranging from 0.001% to 1.0% cross-linking. In such a setup, each biodegradable polymer layer shows a different reaction kinetic with the biomolecules defined above. If the cross-linking range is low, the polymer layer is typically destroyed fast resulting in an early colour change. If the cross-linking range is high, the polymer layer is typically degraded much later due to higher resistance to enzymatic and/or chemical reactions and the colour change, therefore, occurs later in time. With a device built in this setup, it is possible to cover different points in time for the reaction of the biomolecules with the biodegradable polymer layer and, eventually, different spoilage-stages can be monitored.

The above-given explanations with respect to different cross-linking ranges of course also apply for a combination of devices comprised of polymer layers of different thicknesses, wherein the degradation correlates with the thickness of each layer as set out above.

Another example for such a combined device is the following embodiment wherein biodegradable polymer layers with different sensitivities for specific enzymes (wherein the sensitivity is obtained by the kind of polymer used) are placed next to each other combined with a reference device in such a way, that one can deduce which enzymes are present. Using such a device, specific enzymes and, therefore, specific microorganisms secreting these enzymes may be determined.

The device according to the invention may also comprise a "support layer". This term is meant to describe a further layer, which is positioned on the reflecting layer on the opposite side of the polymer layer. Said support layer may be implicated and important during the production of the device according to the invention. The support layer may, however, also be part of the packaging material of the natural product, e.g. a PET foil. The support layer is preferably inert to any reaction with the natural product and, furthermore, biomolecules are preferably able to penetrate said layer and contact the next layer, i.e. to the reflecting layer.

As already set out above, the material used in the support layer may also be part of the reflecting layer if needed as support for e.g. reflecting metallic or coloured pigments; in this situation, there is no support layer as just mentioned above comprised in the device, but rather a reflecting layer comprised of e.g. reflecting metallic or coloured pigments and a support material.

The term "reference device" according to the present invention defines a device of a specific colour or a colour range, wherein the colour/colour range is not subjected to a change of colour. To this aim, the reference device is in a preferred embodiment of the invention coloured by any technique known to the person skilled in the art; however, care should be taken when using colouring agents such that the natural product is not exposed to a potentially harmful colouring agent. The reference device may also comprise a polymer layer which is not subject to degradation by biomolecules and thus maintains its optical properties even when exposed to biomolecules.

The reference device may have one colour which is identical to the colour of the device according to the invention in case the biodegradable polymer layer is totally intact. Furthermore, the reference device may have a second colour, which is identical to the device according to the invention in case the biodegradable polymer layer is substantially up to totally degraded by biomolecules as mentioned above. In this embodiment, the consumer is able to compare the colour of the device according to the invention to the two "on" and "off" situations as explained above.

Of course, the reference device may comprise more than one or two colours for comparison reasons. In an also preferred embodiment, the reference device does not display certain specific colours, but is comprised of a non-degradable polymer layer ranging from the thickness of the device of the invention prior to degradation to zero and, therefore, displays a colour range. Again, the consumer may compare the colour of the device according to this invention to said colour range. The reference device may be positioned directly next to the device of the invention. Furthermore, the reference device is preferably inert to any reactions with the natural product.

The device according to the invention may have different forms. In a preferred embodiment of the invention, the form of the device corresponds to the form of the packaging material of the natural product, e.g. by way of integrating the device into the packaging material and/or preferably by a setup wherein the reflecting layer as set out above corresponds to the packaging material. In one embodiment, the support layer as defined above is a PET-foil also used as packaging material.

Therefore, in an especially preferred embodiment of the present invention, the device thus represents a sensor with an extensive area directly integrated into the packaging material. However, the device of the invention may also have the form of a square or a stripe. In other embodiments of the invention, the form of the device may be a circle, rectangle, ellipse or any other suitable form.

Furthermore, the present invention is concerned with methods for preparing the aforementioned devices. In one embodiment, a reflecting layer, e.g. a CrNi-layer is provided.

Onto said reflecting layer, a biodegradable polymer layer, e.g. PLA or preferably PLGA, optionally in combination with a cross-linking agent such as Desmodur, is applied.

In a further preferred embodiment of the invention, a support layer is firstly provided, e.g. a translucent film, such as a PET film. Onto said support layer, a reflecting layer is applied, e.g. a gold layer. Onto said reflecting layer, a biodegradable polymer layer, e.g. PHB, is then applied.

In preferred methods for preparing such devices, the polymer layer may be applied by dip coating or film-printing techniques, such as gravure printing, or by spin coating. Such techniques are routine methods to the skilled person in the art. Any other technique known to the person skilled in the art leading to the application of thin polymer layers onto other layers may also be used. In preferred embodiments, PLGA is used as material for the polymer layer. In the methods for preparing the polymer layer, PLGA may be used in a concentration (weight/volume) ranging from about 19% w/v to about 35% w/v in a suitable solvent, such as trifluorethanole. The concentration of the cross-linking agent used is also critical for the method of preparing the polymer layer. Desmodur might be used as cross-linking agent in a concentration (volume/volume) ranging from about 10⁻⁹% v/v to about 5.0% v/v.

In still other embodiments of the invention, methods for analyzing the age and/or quality of a natural product are provided.

In one aspect of the invention, the natural product is contacted with the reflecting layer and/or the support layer, if present. Thus, the biomolecules as defined above are able to penetrate the reflecting layer and/or the support layer, if present, and contact the biodegradable polymer layer.

In this embodiment, one may directly determine the colour of the device, e.g. by looking at the device from the side of the polymer layer.

In another aspect of the invention, the natural product is contacted directly with the biodegradable polymer layer of the device; thus, the biomolecules may act directly on the polymer without the need of penetrating a layer. In order to analyze the colour of the device, it may be necessary to remove the natural product from the device prior to the analysis.

The method for analyzing the age and/or quality of a natural product may furthermore comprise the comparison of the colour of the device to the colour of a reference device as already mentioned above.

In other embodiments, the present invention is concerned with the use of a device according to the present invention for the analysis of the age and/or quality of a natural product. Due to the correlation of the presence of spoilage-causing microorganisms and the colour of the device (via degrading enzymes of the microorganisms acting on the polymer layer resulting in a colour change as set out above), it is possible to carry out such an analysis.

Thus, one may use a device according to the invention for analyzing the quality of a natural product as defined above, i.e. whether a cosmetic product is still applicable to the human body, whether a pharmaceutical product may still be used or whether a food is still edible.

A device according to the invention may also be used in order to analyze if the production steps and/or storage of medical products, cosmetics or foods have been handled correctly.

Since most of the microorganisms responsible for the spoilage preferably proliferate at 37°C, natural products are stored and transported at temperatures below 37°C, preferably at 4°C or even frozen at -20°C to maintain an unfavourable temperature range for such microorganisms. Failure to comply with said conditions may result in contamination, which can be analyzed for by the present device.

In the following, some embodiments in accordance with the invention are illustrated by way of examples. These examples are, however, not to be construed as limiting.

### EXAMPLES

Example 1: Incubation of a device according to the invention with degrading enzymes in different concentrations results in a colour change.

### Preparation of the device

A NiCr-alloy (Inconnel by CPFilms, OD 2.2) was used as reflective layer. Onto this layer, a polymer layer of 21 % (by weight) PLGA [poly (D,L-lactic-co-glycolic acid] and 0.5 % (by weight) Desmodur in ethylacetate was printed (parameters: "feine Druckplatte" by Hueck, "Anpressdruck: Walze + 15, Rakelmesser 0", velocity of 5, followed by an incubation for 10 minutes by 80°C).

### Incubation of the device

An enzyme mix of the proteases proteinase K, trypsin and chymotrypsin (in a ratio of 1:1:1) was prepared in 0.1 M Tris/HCl, pH 8.2 to a final protein concentration of 20 mg/ml. Starting from this solution, different concentrations as indicated below were obtained by corresponding dilution steps in 0.1 M Tris/HCl, pH 8.2.

The proteases (as well as Tris-buffer and water as negative controls) were pipetted onto the biodegradable polymer layer in different concentrations as indicated below. After an incubation of 4 h at 4 °C, the device was washed with ddH₂O, dried under an airstream and scanned from the polymer layer-side to make the color change visible (Figure 2).

### Pipetting scheme:

| | Left side | Right side |
|---|---|---|
| 1 | Enzyme mix, 20 mg/ml | Enzyme mix, 5 mg/ml |
| 2 | Enzyme mix, 15 mg/ml | Enzyme mix, 2.5 mg/ml |
| 3 | Enzyme mix, 12 mg/ml | 0.1 M Tris/HCl, pH 8.2-buffer |
| 4 | Enzyme mix, 10 mg/ml | H₂O |

The incubation with the proteases resulted in a colour change as clearly visible in Fig. 2, whereas the incubation with buffer or water did not lead to a change in the colour. The change of the colour correlated with the concentration of the enzyme mix; thus, incubation with an enzyme mix at lower concentrations (2.5 and 5 mg/ml, see right side, spots 1 and 2) resulted in a different colour than the incubation with an enzyme mix at higher concentrations (see left side).

### Example 2: Sensor for detecting meat juice, bacteria, a contaminated solution for contact lenses and contaminated hand cream.

The general protocol for preparing the devices according to the invention was: A NiCr-alloy (Inconnel by CPFilms, OD 2.2) was used as reflective layer. Onto this layer, a polymer layer of PLGA with weight-percent as indicated below and Desmodur with weight-percent as indicated below in ethylacetate was printed (parameters: "feine Druckplatte" by Hueck, "Anpressdruck: Walze + 15, Rakelmesser 0", velocity of 5, followed by an incubation for 10 minutes by 80°C).

The following devices according to the invention were prepared and tested for their ability to detect the above mentioned samples:
A) 21% PLGA with 0.5% Desmodur
   420 mg PLGA and 200 µl 100% Desmodur were mixed in 3.8 ml ethylacetate and allowed to react at room temperature under stirring.
B) 21% PLGA with 1.0% Desmodur
   420 mg PLGA and 400 µl 100% Desmodur were mixed in 3.6 ml ethylacetate and allowed to react at room temperature under stirring.
C) 23% PLGA with 5 x 10⁻⁸ % Desmodur
   420 mg PLGA and 1 µl of a 10⁻⁴ % Desmodur solution were mixed in 3.99 ml ethylacetate and allowed to react at room temperature under stirring.

The devices were then incubated with meat juice (mj), meat homogenate (mh), bacteria (bac), contaminated solution for contact lenses (cl) and contaminated hand cream (hc), wherein the samples were directly pipetted onto the polymer layer.

### Preparation of the samples:

### Preparation of meat juice and the bacterial cocktail D

In order to prepare meat juice (from pork meat, bought as packaged meat in a supermarket), the meat was homogenised in 20 g portions with 180 ml of a standard peptone-glycerol-buffer. The meat juice was then aliquoted in 1 ml aliquots and either directly used or stored in 100 µl aliquots at -80°C.

In order to induce growth of spoiling bacteria in the meat juice, the meat juice was incubated for 5 h at 37°C. The contaminated meat juice (corresponding to the sample "mj" as mentioned above) was then incubated on media plates (GSP- and ÖNÖZ Salmonella-Agar) at 37°C. Following this incubation, the colonies of the aerobe gram negative bacteria of the strains *Pseudomonas, Enterobacter, Proteus* and *Salmonella* were identified according to their morphology, and picked.

For the cocktail, 3 ml selective liquid media were inoculated with the four strains mentioned above and incubated in at 37°C for 24 h under shaking at 180 rpm. Following this incubation, 0.5 ml of this culture were used to inoculate 100 ml liquid media with phosphate (phosphate was used in order to induce the excretion of enzymes). The bacteria were then incubated at 30°C for 16 h (180 rpm). Following this incubation, the bacterial cocktail was aliquoted in 1 ml aliquots and frozen in liquid nitrogen and stored at -80°C, representing bacterial cocktail D.

### Preparation of the meat homogenate

Meat samples (pork cutlets) were supplied from the local supermarket (Spar). Meat samples of 10 g were placed in Petri dishes, infected with bacterial cocktail D (prepared as outlined above, used in bacterial concentration of 1000 CFU/g) and incubated as follows: For meat homogenate I (mh I), the samples were incubated for 96 hours at 4 °C, and for homogenate II (mh II), the samples were incubated for 48 hours at 15°C.

The contaminated meat was then transferred into 90 ml liquid media in Stomacher Bags and homogenized using a Stomacher® 400 Circulator, 1 min by 230 rpm.

The homogenates were then aliquoted in 1 ml aliquots, frozen in liquid nitrogen and stored at - 80 °C.

### Preparation of the contaminated pharmaceutical product

50 µl of both, *Pseudomonas* and *Enterobacter* strains (grown separately in liquid media as mentioned above) were added to 50 ml of Acumed, a solution for contact lenses (4Care AG, Kiel, Germany). The resulting contaminated solution was incubated for 24 h at room temperate corresponding to the sample "contaminated solution for contact lenses" as mentioned above.

### Preparation of the contaminated cosmetic

10 g of "hand & nail cream Kamill" (Burnus, Darmstadt, Germany) were mixed with 50 ml Ringer-solution and thoroughly vortexted. To the resulting suspension, 50 µl of both, Pseudomonas and Enterobacter strains (grown separately in liquid media as mentioned above) were added. The resulting contaminated suspension was incubated for 24 h at room temperate corresponding to the sample "contaminated hand cream" as mentioned above.

### Determination of the titer

For each sample, the titer of the microorganisms were determined according to standard microbiological tests and calculated as number of colony forming units (CFU) either per gram in case of meat juice (mj) and meat homogenate (mh) or per ml in case of solutions (bacterial cocktail D (bac), Acumed (cl) and hand cream (hc)).

Following the tests, the samples were then pipetted onto the devices according to the following scheme:

| | row 1 | row 2 | row 3 |
|---|---|---|---|
| 1 | mj, 10⁶ CFU/g | | bac, 10⁷ CFU/ml |
| 2 | mj, 10⁷ CFU/g | mh I, 10⁷ CFU/g | bac, 10⁶ CFU/ml |
| 3 | cl, 10⁵ CFU/ml | mh II, 10⁸ CFU/g | Phospholipase (10mg/ml) |
| 4 | hc, 10⁶ CFU/ml | mh II, 10⁶ CFU/g | Ringer-solution |

Finally, the devices were incubated at 37°C for 6 hours and then scanned for documentation of the colour changes.

Phospholipase was used as positive control and resulted in a colour change in all devices, whereas the negative control Ringer-solution did not lead to a change in the colour of any of the devices.

A concentration dependent effect could be observed for the colour change upon the incubation with bacterial cocktail D; A higher concentration seems to result in a more pronounced colour change, i.e. towards a brighter colour.

A concentration dependent effect could also be observed for the colour change upon incubation with meat homogenate. A CFU of 10^{g} / g seems to result in brighter colour compared to the sample comprising 10⁷ CFU / g.

The brightest colour (corresponding to the most pronounced colour change) could be observed upon the incubation with contaminated hand crème; however, incubation with the contaminated solution for contact lenses also resulted in a clearly visible colour change.

The incubation with the meat juice-samples also clearly resulted in colour changes of all three devices.

In general, the use of devices with 21% PLGA seems to result in more pronounced colour changes.

## Claims

1. A device comprising a biodegradable polymer layer positioned on a reflecting layer, wherein the device is configured such that biomolecules capable of degrading said polymer layer are allowed to contact said polymer layer, wherein the device is configured such that degradation of said polymer layer results in a colour change, and wherein said device comprises only one reflecting layer.

2. A device according to claim 1 wherein said polymer layer does not comprise a colouring agent.

3. A device according to any of the preceding claims wherein said polymer layer is selected from a polymer comprising comprising poly(lactic-*co-*glycolic acid), polylactic acid, poly-L-lactic acid, polyhydroxybutyrate and polyvinylcaprolactame.

4. A device according to any of the preceding claims wherein said reflecting layer is selected from a material comprising a metal, preferably gold, titan or aluminium; an alloy, preferably CrNi or TiAl; silicium; a reflecting oxide, preferably Eloxal; glassy carbon; tin; Geberit; opaque reflecting glass; a lacquer; and a metallic or coloured pigment within a support.

5. A device according to any of the preceding claims wherein said reflecting layer is selected from a material consisting of glassy carbon or silicium.

6. A device according to any of the preceding claims wherein said reflecting layer corresponds to or is part of the packaging material of said natural product.

7. A device according to any of the preceding claims wherein biomolecules are allowed to penetrate said reflecting layer in order to contact said polymer layer.

8. A device according to any of the preceding claims wherein the device also comprises a reference device.

9. A method for preparing a device according to any of claims 1 to 8 wherein said method comprises at least the steps of
a) Providing a reflecting layer, and
b) Applying a biodegradable polymer layer onto said reflecting layer.

10. A method according to claim 9 wherein said polymer layer is applied by dip coating or film-printing.

11. A method for analyzing the age and/or quality of a natural product comprising cosmetics, pharmaceutical products and foods which comprises at least the following steps:
a) Providing a device according to any of claims 1 to 8;
b) Contacting said device with said natural product;
c) Determining the colour of said device;
d) Comparing the colour of said device to the colour of a reference device;
e) Determining the age and/or quality of said natural product according to this comparison.

12. A method for analyzing the age and/or quality of a natural product according to claim 11 wherein said polymer layer of said device is being contacted in step b) directly with said natural product.

13. A method for analyzing the age and/or quality of a natural product according to claim 11 wherein the reflecting layer and/or the support layer of said device is/are being contacted in step b) with said natural product such that biomolecules are allowed to penetrate the reflecting layer and/or the support layer in order to contact said polymer layer.

14. The use of a device according to any of claims 1 to 8 for the analysis of the age and/or quality of a natural product comprising cosmetics, pharmaceutical products and foods.

15. The use of a device according to claim 14 for the analysis of the age and/or quality of a natural product by detecting enzymes and/or catabolic metabolites of microorganisms and/or of the natural product via the degradation of said polymer layer.
